# EUROPEAN PATENT APPLICATION

(11) **EP 4 847 518 A1**
(43) Date of publication of application: **02.12.2026**
(21) Application number: 24920103.9
(22) Date of filing: 30.10.2024
(51) Int. Cl.: C07F 15/06, C07B 61/00, C07C 49/92, C07C 211/05, C07C 211/48, C07C 211/65, C07C 215/08, C07C 235/74, C07C 235/80, C07D 207/06, C07D 213/06, C07D 213/22, C07D 295/185, C07D 307/33

(54) **METHOD FOR SEARCHING FOR LIGAND**

(30) Priority: 24.01.2024 JP 2024008833; 22.03.2024 JP 2024045903
(71) Applicant: Resonac Corporation, Tokyo 105-7325 (JP)
(72) Inventor: NAKAJIN, Kokin, Tokyo 105-7325 (JP); NISHIZAWA, Shohei, Tokyo 105-7325 (JP); USAMI, Akira, Tokyo 105-7325 (JP)
(74) Representative: Strehl & Partner mbB
(86) International application number: PCT/JP2024/038755
(87) International publication number: WO 2025/158745

(57) **Abstract**

One embodiment of the present invention is a method for searching for a ligand to be coordinated to cobalt, and the method includes causing a processor to execute processes including searching for a ligand that is any of ligands represented by general formulae (I) to (VI), the ligand being a ligand with which a logarithmic value of a predicted curing time [T] [sec] calculated by an equation (1) below is equal to or lower than a predetermined value, ${log}_{10} \left[T\right] = {α}_{0} + {α}_{1} {x}_{1} + {α}_{2} {x}_{2}$ (1) (in the equation, x₁ is an NBO charge of a cobalt atom in a dormant species of a complex in which the ligand is coordinated to the cobalt atom, x₂ is percent buried volume of the ligand present within 3 Å of the cobalt atom in the dormant species of the complex in which the ligand is coordinated to the cobalt atom, a₀, and a₁, and a₂ are coefficients).

## Description

### TECHNICAL FIELD

The present invention relates to ligand search methods.

### BACKGROUND ART

Conventionally, when a radically polymerizable resin, such as an unsaturated polyester resin, a vinyl ester resin, or the like, is cured at room temperature, a redox initiator including a curing agent and a curing accelerator is generally used. As a combination of the curing agent and the curing accelerator (curing agent/curing accelerator) included in the redox initiator, for example, a combination of ketone peroxide and a cobalt organic acid salt (ketone peroxide/cobalt organic acid salt) has been known.

By adding a ligand to the cobalt organic acid salt, the curing performance of the cobalt organic acid salt used as the curing accelerator changes, thereby changing the curing performance of the redox initiator. Therefore, in order to cure a radically polymerizable resin at room temperature in a short time, a method for searching for a ligand to be included in a curing accelerator capable of shortening a curing time of the radical polymerizable resin has been studied.

As the method for searching for a ligand to be added to a curing accelerator, for example, there is disclosed a method for searching for a ligand, which includes a step of searching for a ligand, such as substituted aniline or the like, with which a curing time calculated by a linear regression equation becomes equal to or shorter than a predetermined time, and the linear regression equation represents a relationship of a LUMO level and a HOMO level of a dormant species of a complex in which the ligand is coordinated, and oxygen atoms present within 2 [Å] of the cobalt atom (see, for example, Patent Document 1).

### RELATED ART DOCUMENTS

### PATENT DOCUMENTS

Patent Document 1: Japanese Unexamined Patent Application Publication No. 2021-147350

### DISCLOSURE OF THE INVENTION

### PROBLEMS TO BE SOLVED BY THE INVENTION

For example, in order to cure a radically polymerizable resin at room temperature in a shorter time using a redox initiator, it is important to further improve prediction accuracy of a curing time of the radically polymerizable resin when a different kind of a curing accelerator is used in the redox initiator. In the method for searching for the ligand of Patent Literature 1, values associated with electronic characteristics of a molecule constituting the curing accelerator are used as physical property values for predicting a curing time of the radically polymerizable resin, and steric properties are not taken into consideration.

One aspect of the present invention aims to provide a ligand search method that highly accurately searches for a ligand of a curing accelerator capable of shortening a curing time when a radically polymerizable resin is cured at room temperature.

### MEANS FOR SOLVING THE PROBLEMS

The present invention includes the following configurations.
[1] A method for searching for a ligand to be coordinated to cobalt, the method comprising:
   causing a processor to execute processes including
   searching for a ligand that is any of
      a ligand represented by a general formula (I): 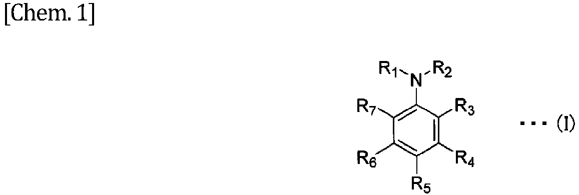 (in the formula, R₁ and R₂ are each independently a hydrogen atom or an alkyl group with 1 to 10 carbon atoms, and R₃ to R₇ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, or a halogen atom),
      a ligand represented by a general formula (II): 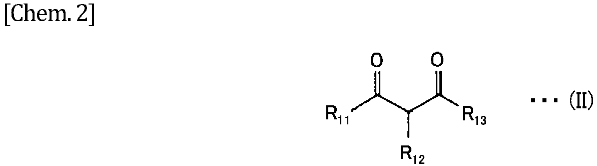 (in the formula, R₁₁ to R₁₃ are each independently a hydrogen atom, an alkyl group with 1 to 10 carbon atoms, an alkoxy group with 1 to 10 carbon atoms, an aryl group with 6 to 12 carbon atoms, an amino group, a carboxy group, a cyano group, or a halogen atom, and R₁₂ and R₁₃ may form a ring together),
      a ligand represented by a general formula (III): 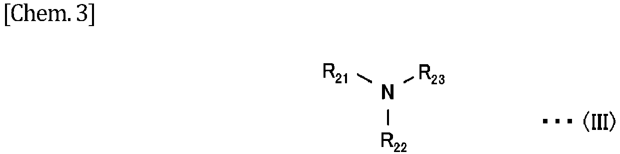 (in the formula, R₂₁ to R₂₃ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, or a halogen atom), and
      a ligand represented by a general formula (IV): 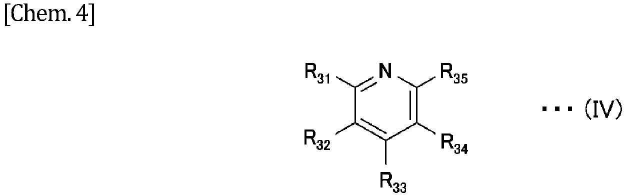 (in the formula, R₃₁ to R₃₅ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, a halogen atom, or a pyridine ring),
         the ligand being a ligand with which a logarithmic value of a predicted curing time [T] [sec] calculated by an equation (1) below is equal to or lower than a predetermined value, ${log}_{10} \left[T\right] = {α}_{0} + {α}_{1} {x}_{1} + {α}_{2} {x}_{2}$ (1) (in the equation, x₁ is an NBO charge of a cobalt atom in a dormant species of a complex in which the ligand is coordinated to the cobalt atom, x₂ is percent buried volume of the ligand present within 3 Å of the cobalt atom in the dormant species of the complex in which the ligand is coordinated to the cobalt atom, a₀, and a₁, and a₂ are coefficients).
[2] The method for searching for the ligand according to [1],
   wherein the equation (1) is an equation (2): ${log}_{10} \left[T\right] \left({x}_{i}\right) = 5.20950 {x}_{1} - 0.50360 {x}_{2} + 47.001$ (2), and
   the predetermined value is 5.0.
[3] The method for searching for the ligand according to [1] or [2],
   wherein the dormant species of the complex in which the ligand represented by the general formula (I), (III), or (IV) is coordinated to the cobalt is,
   on a basis of a cobalt (II) Werner-type complex in which two molecules of a carboxylate ligand as a bidentate ligand and two molecules of the ligand represented by the general formula (I), (III), or (IV) as a monodentate ligand are coordinated,
   a dormant species of the Werner-type complex with cobalt (III), in which an OH radical or Me radical is coordinated instead of one of the two molecules of the ligand represented by the general formula (I), (III), or (IV).
[4] The method for searching for the ligand according to any one of [1] to [3],
   wherein the dormant species of the complex in which the ligand represented by the general formula (II) is coordinated to the cobalt is,
   on a basis of a cobalt (III) Werner-type complex in which three molecules of the ligand represented by the general formula (II) as a bidentate ligand is coordinated,
   a dormant species of the cobalt (III) Werner-type complex, in which a OH radical or Me radical is coordinated instead of one of the three molecules of the ligand represented by the general formula (II).

### EFFECTS OF THE INVENTION

According to one aspect of the present invention, there can be provided a ligand search method that highly accurately searches for a ligand of a curing accelerator capable of shortening a curing time when a radically polymerizable resin is cured at room temperature.

### BRIEF DESCRIPTION OF THE DRAWINGS

[Fig. 1] Fig. 1 is a schematic view illustrating one example of a ligand search system.
[Fig. 2] Fig. 2 is a block diagram illustrating a functional configuration of a regression model generation device.
[Fig. 3] Fig. 3 is a diagram illustrating one example of a correlation between a measured curing time and a predicted curing time.
[Fig. 4] Fig. 4 is a block diagram illustrating a functional configuration of a ligand search device.
[Fig. 5] Fig. 5 is a block diagram illustrating a hardware configuration of the regression model generation device and the ligand search device.
[Fig. 6] Fig. 6 is a flowchart that explains the ligand search method according to the present embodiment.
[Fig. 7] Fig. 7 is a flowchart illustrating a regression model generation process.
[Fig. 8] Fig. 8 is a flowchart illustrating a ligand search process.
[Fig. 9] Fig. 9 is a diagram illustrating the correlation between the logarithmic value of the predicted curing time and the logarithmic value of the measured curing time when the ligands of Reference Examples 1 to 13 are used.
[Fig. 10A] Fig. 10A is a diagram illustrating plotted positions of the correlation between the logarithmic value of the predicted curing time and the logarithmic value of the measured curing time when the ligand of Reference Example 5 is used as test data.
[Fig. 10B] Fig. 10B is a diagram illustrating plotted positions of the correlation between the logarithmic value of the predicted curing time and the logarithmic value of the measured curing time when the ligand of Reference Example 7 is used as test data.
[Fig. 10C] Fig. 10C is a diagram illustrating plotted positions of the correlation between the logarithmic value of the predicted curing time and the logarithmic value of the measured curing time when the ligand of Reference Example 8 is used as test data.

### MODE OF CARRYING OUT THE INVENTION

Hereinafter, embodiments of the present invention will be described in detail. In the present specification, "to" indicating a numerical range means that numerical values described before and after the "to" are included as a lower limit value and an upper limit value, respectively, unless otherwise specified.

The ligand search method according to the present embodiment will be described. The ligand search method according to the present embodiment is a method for searching for a ligand of a curing accelerator to be included in a curing agent. For describing the ligand search method according to the present embodiment, a ligand search system used in the ligand search method according to the present embodiment will be described.

### <Ligand search system>

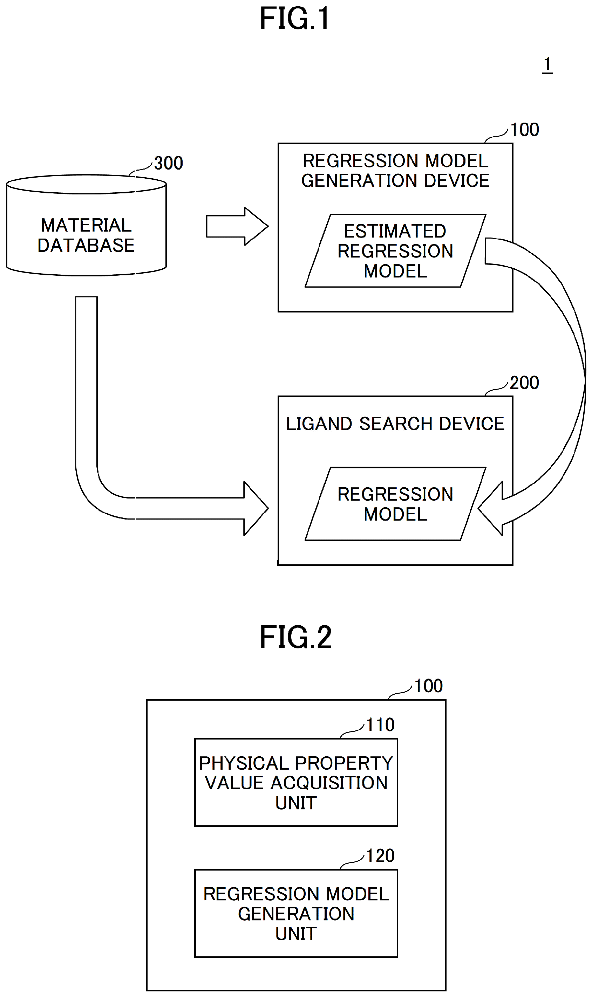

An outline of the ligand search system used in the ligand search method according to the present embodiment will be described. Fig. 1 is a schematic view illustrating one example of the ligand search system. As illustrated in Fig. 1, the ligand search system 1 includes a regression model generation device 100 and a ligand search device 200. In the ligand search system 1, the regression model generation device 100 acquires material data (e.g., a structure of a ligand of a curing accelerator, physical property values, a logarithmic value of a curing time, or the like) regarding the curing accelerator from material database 300, and constructs a regression model for predicting a logarithmic value of a curing time from the physical property values of the curing accelerator. In the ligand search system 1, the ligand search device 200 searches for a ligand of the curing accelerator with which a logarithmic value (log₁₀ [T]) of a predicted curing time [T] [sec] of a redox initiator including the curing accelerator becomes equal to or lower than a predicted value, where the predicted curing time [T] [sec] is calculated by the following equation (1) from the physical property values of the curing accelerator including any of ligands represented by the following general formulae (I) to (IV) using the generated regression model. ${log}_{10} \left[T\right] = {α}_{0} + {α}_{1} {x}_{1} + {α}_{2} {x}_{2}$ (1)
(in the equation, x₁ is an NBO charge of a cobalt atom in a dormant species of a complex in which the ligand is coordinated to the cobalt atom, x₂ is percent buried volume of the ligand present of 3 Å from the cobalt atom in the dormant species of the complex in which the ligand is coordinated to the cobalt atom, a₀, and a₁, and a₂ are coefficients.) 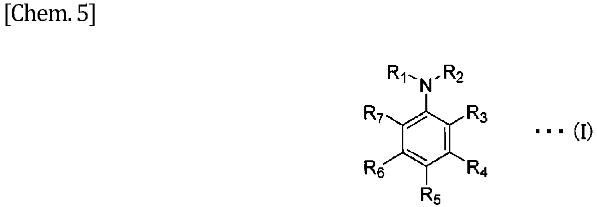
(In the formula, R₁ and R₂ are each independently a hydrogen atom or an alkyl group with 1 to 10 carbon atoms, and R₃ to R₇ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, or a halogen atom.) 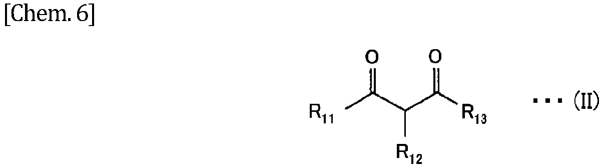
(In the formula, R₁₁ to R₁₃ are each independently a hydrogen atom, an alkyl group with 1 to 10 carbon atoms, an alkoxy group with 1 to 10 carbon atoms, an aryl group with 6 to 12 carbon atoms, an amino group, a carboxy group, a cyano group, or a halogen atom, and R₁₂ and R₁₃ may form a ring together.) 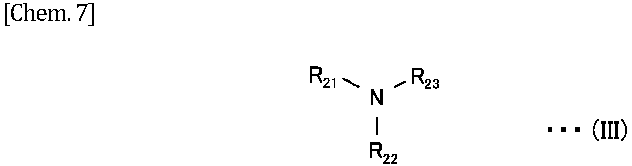
(In the formula, R₂₁ to R₂₃ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, or a halogen atom.) 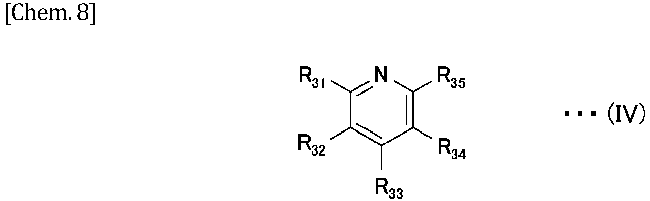
(In the formula, R₃₁ to R₃₅ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, a halogen atom, or a pyridine ring.)

The amino group in the general formulae (I) to (IV) includes a primary amino group (-NH₂), a secondary amino group (-NHR), or a tertiary amino group (-NR₂).

R in the general formulae (I) to (IV) is preferably an alkyl group with 1 to 10 carbon atoms.

In the case where the amino group in the general formulae (I) to (IV) is a tertiary amino group, two Rs in the tertiary amino group may be the same or different, and may form a ring together.

Examples of a halogen atom as each of Rs in the general formulae (I) to (IV) include a chlorine atom, a bromine atom, an iodine atom, and the like.

The NBO charge is a charge determined by a natural bonding orbit (NBO) of each atom. The natural electron density of each atom is obtained by performing a natural bonding orbital (NBO) analysis on the electronic state calculated by a calculation (e.g., B3LYP/6-31G* or the like) in which a calculation method, such as density-functional theory (DFT) or the like, and a basis function are combined. The sum of the natural electron density and the atomic number corresponding to the positive charge density for each atom is the NBO charge. The NBO charge is an index indicating an electrical bias in each atom.

The dormant species of the complex in which the ligand is coordinated to the cobalt is generated by coordinating an active pieces (P·) to cobalt constituting a Werner-type complex when a radically polymerizable resin is cured at room temperature using a redox initiator including ketone peroxide and the cobalt organic acid salt.

Examples of the active species (P·) include a radical generated by decomposition of the ketone peroxide, a radical generated by a reaction between the radical generated by decomposition of the ketone peroxide and the radically polymerizable resin, and the like.

The Werner-type complex is an octahedral complex in which ligands each having an unshared electron pair are coordinated to a central metal ion.

In the present embodiment, as the Werner-type complex, a cobalt (II) complex in which two molecules of a carboxylate ligand as a bidentate ligand and two molecules of the ligand represented by the general formula (I), (III), or (IV) as a monodentate ligand are coordinated, or a cobalt (II) complex in which three molecules of the ligand represented by the general formula (II) as a bidentate ligand are coordinated is used.

In the dormant species of the complex in which the ligand is coordinated to the cobalt, an OH radical (·OH), a Me radical (·Me), or the like may be coordinated instead of the active species (P·) included in the dormant species. As the Me radical, for example, a polymer chain grown by increasing carbons with Me, Et, or the like, may be used.

Specifically, on the basis of the cobalt (II) Werner-type complex in which two molecules of a carboxylate ligand as a bidentate ligand and two molecules of the ligand represented by the general formula (I), (III), or (IV) as a monodentate ligand are coordinated, the dormant species of the complex in which the ligand represented by the general formula (I), (III), or (IV) is coordinated to cobalt may be a geometry-optimized dormant species of the Werner-type complex with a cobalt (III), in which one molecule of an OH radical, a Me radical, or the like is coordinated instead of one of the two molecules of the ligand represented by the general formula (I), (III), or (IV).

The dormant species of the complex in which the ligand represented by the general formula (II) is coordinated to cobalt may be, on a basis of a cobalt (III) Werner-type complex in which three molecules of the ligand represented by the general formula (II) as a bidentate ligand is coordinated, a geometry-optimized dormant of the cobalt (III) Werner-type complex, in which one molecule of an OH radical, a Me radical, or the like is coordinated instead of one of the three molecules of the ligand represented by the general formula (II).

Examples of the carboxylate ligand include an acetato ligand and the like.

A cobalt complex, such as a complex in which the above ligand is coordinated to cobalt, is generally capable of curing a radically polymerizable resin at room temperature through a chain transfer mechanism. For example, in the cobalt (II) Werner-type complex in which two molecules of the carboxylate ligand as a bidentate ligand and two molecules of the ligand represented by the general formula (I), (III), or (IV) as a monodentate ligand are coordinated, one molecule of an active species (P₁·) is coordinated instead of one of the two molecules of the ligand represented by the general formula (I), (III), or (IV), thereby forming a complex of cobalt (III), i.e., a dormant species. As in the following formula (a), the dormant species exchanges the active species (P₁·) with another active species (P₂·), thereby releasing the active species (P₁·). The active species (P_{1·}) reacts with an unsaturated unit of a polymer chain of a radically polymerizable resin, thereby forming an active species, and the obtained active species reacts with the cobalt complex, thereby forming a dormant species of the cobalt complex, from which the active species (P₂·) is released.

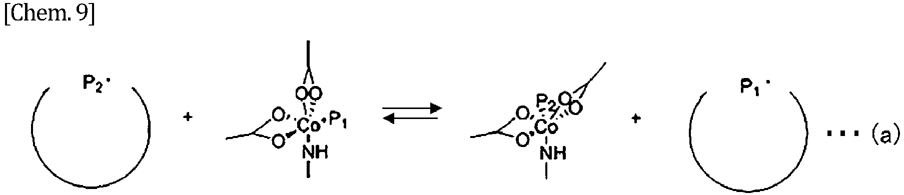

In addition, in the cobalt (II) Werner-type complex of cobalt in which three molecules of the ligand represented by the general formula(II) as a bidentate ligand are coordinated, one molecule of an active species (P_{1·}) is coordinated instead of one of the three molecules of the ligand represented by the general formula (II), thereby forming a complex of cobalt (III), i.e., a dormant species. As in the following formula (b), the dormant species exchanges the active species (P₁·) with another active species (P₂·) to release the active species (P₁·). The active species (P_{1·}) reacts with an unsaturated unit of a polymer chain of a radically polymerizable resin, thereby forming an active species, and the obtained active species reacts with a cobalt complex, forming a dormant species of the cobalt complex, from which the active species (P₂·) is released.

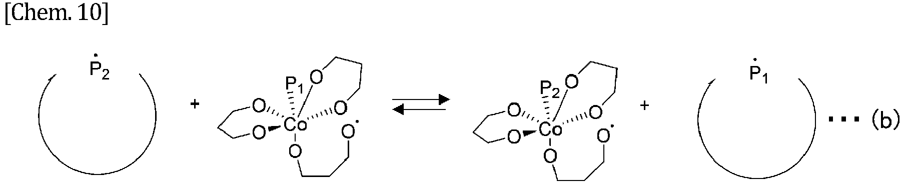

In such a chain transfer mechanism in which the activation reaction and the deactivation reaction simultaneously occur, the overall reaction rate is equal to the exchange reaction rate. The higher the exchange reaction rate is, the more curing of the radically polymerizable resin proceeds.

The ligand search system 1 searches for a ligand of a curing accelerator used when a radically polymerizable resin is cured at room temperature, with reference to the value of the log₁₀ [T] determined using the regression model generated by the regression model generation device 100. As in the equation (1), the regression model is represented by a simple linear regression equation using two physical property values xᵢ (i = 1 and 2), which include an NBO charge x₁ of the cobalt atom in the dormant species of the complex in which the ligand is coordinated to the cobalt atom, and the percent buried volume x₂ of the ligand present within 3 Å of the cobalt atom in the dormant species of the complex in which the ligand is coordinated to the cobalt atom. The reduction of the log₁₀ [T] calculated by the linear regression equation of the equation (1) corresponds to shortening of a curing time T (hereinafter, referred to as a measured curing time T) actually measured using the curing accelerator including the cobalt organic acid salt and the ligand. Therefore, the ligand search system 1 can highly accurately search for a ligand of a curing accelerator capable of curing a radically polymerizable resin at room temperature in a short time.

### [Regression model generation device]

The regression model generation device 100 will be described. Fig. 2 is a block diagram illustrating a functional configuration of the regression model generation device 100. As illustrated in Fig. 2, the regression model generation device 100 includes a physical property value acquisition unit 110 and a regression model generation unit 120. The regression model generation device 100 acquires material data (e.g., a curing time) regarding a curing accelerator from the material database 300, and generates a regression model for predicting a curing time from the physical property values of the curing accelerator. The regression model generation device 100 provides the generated regression model to the ligand search device 200.

### (Physical property value acquisition unit)

The physical property value acquisition unit 110 acquires material data used for generating a regression model from the material database 300, and executes predetermined processing, such as quantum chemical calculations or the like, on the acquired material data, thereby acquiring physical property values of the material.

The physical property value acquisition unit 110 determines, using for example quantum chemical calculation software, the NBO charge of the cobalt atom in the dormant species of the complex in which a ligand is coordinated to the cobalt atom, and the percent buried volume of the ligand present within 3 Å of the cobalt atom in the dormant species of the complex in which the ligand is coordinated to the cobalt atom.

In order to simplify the calculations, a Me radical (·Me), an OH radical (·OH), or the like may be used instead of the active species (P·) coordinated in the dormant species.

As the quantum chemical calculation software, for example, Gaussian 09 available from Gaussian, Inc. or the like may be used. Gaussian 09 may use RωB97XD as a functional and 6 - 31⁺G(d) as a basis function based on the unrestricted density-functional theory. The frequency analysis is performed in all calculations to confirm that the number of the imaginary vibration frequencies is zero, thereby determining that the calculated structures are converged at the bottom of the potential energy curve.

The physical property value acquisition unit 110 may acquire an unoptimized structure or an optimized structure from the material database 300.

When a ligand to be coordinated to cobalt is searched, a cobalt (II) Werner-type complex, in which two molecules of a carboxylate ligand as a bidentate ligand and one molecule of a ligand (L), which is a monodentate ligand or a bidentate ligand different from the carboxylate ligand are coordinated, is used. The ligand is classified into two types, for example, an amine-based ligand substituted with an amine-based partial structure, and a diketone-based ligand substituted with a diketone-based partial structure.

In the case where the ligand (L) is the amine-based ligand substituted with the amine-based partial structure, the ligand (L) is represented by the following chemical formula (A). The chemical formula (A) corresponds to the general formula (I), (III), or (IV) that is a monodentate ligand.

Specifically, in the case where the ligand (L) is the ligand represented by the general formula (I), (III), or (IV) that is a monodentate ligand, a cobalt (II) Werner-type complex, in which two molecules of a carboxylate ligand as a bidentate ligand and the ligand represented by the general formula (I), (III), or (IV) as a monodentate ligand are coordinated is used, when the ligand to be coordinated to cobalt is searched. On the bases of this Werner-type complex, a dormant species of the Werner-type complex with cobalt (III), in which one molecule of an active species (P·) is coordinated instead of one molecule of the ligand (L) represented by the general formula (I), (III), or (IV), is determined as an initial structure of the dormant species in which the ligand represented by the general formula (I), (III), or (IV) is coordinated to the cobalt organic acid salt. In order to simplify the calculations, the initial structure of the dormant species obtained by using a Me radical (·Me) instead of the active species (P·) and approximating the carboxylate ligand with an acetato ligand having a short alkyl chain may be used as the structure represented by the following chemical formula (A).

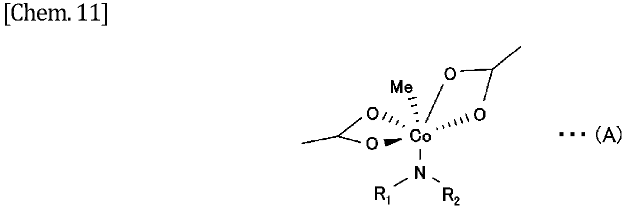

In the case where the ligand (L) is the diketone-based ligand substituted with the diketone partial structure, the ligand (L) is represented by the following chemical formula (B). The chemical formula (B) corresponds to the general formula (II) that is a bidentate ligand.

Specifically, in the case where the ligand (L) includes three molecules of the ligand represented by the general formula (II) as a bidentate ligand, a cobalt (II) Werner-type complex, in which three molecules of the ligand represented by the general formula (II) as a bidentate ligand are coordinated, is used, when a ligand to be coordinated to cobalt is searched. On the bases of this Werner-type complex, a dormant species of the Werner-type complex with cobalt (III), in which one molecule of an active species (P·) is coordinated instead of one of the three molecules of the ligand represented by the general formula (II) is determined as an initial structure of the dormant species in which the ligand represented by the general formula (II) is coordinated to the cobalt organic acid salt. In order to simplify calculations, an initial structure of the dormant species obtained by using the dormant species in which a Me radical (·Me) is coordinated instead of the active species (P·) may be used as the structure represented by the chemical formula (B).

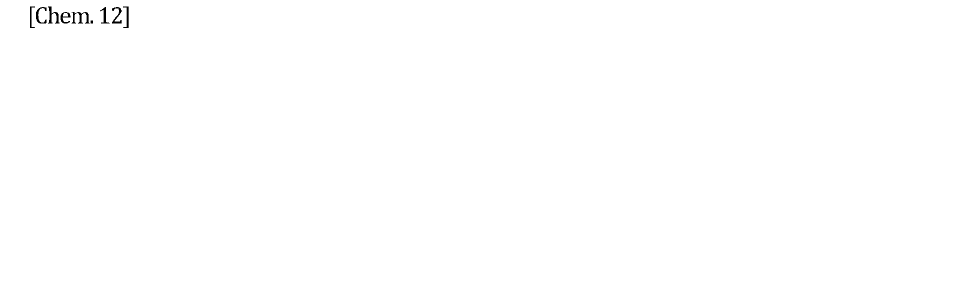

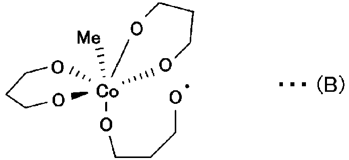

The physical property value acquisition unit 110 preferably performs geometry optimization on the initial structure of the dormant species. A regression model for estimation is assumed to be the following equation (1a) that is a linear regression equation represented by the linear combination of two physical property values xᵢ (i is 0, 1, or 2), and multiple linear regression analysis is performed to determine a coefficient aᵢ (i is 0, 1, or 2). ${log}_{10} \left[T\right] = {α}_{0} + {α}_{1} {x}_{1} + {α}_{2} {x}_{2}$ (1a) (In the equation, x₁ the NBO charge of the cobalt atom in the dormant species of the complex in which the ligand is coordinated to the cobalt atom, x₂ is the percent buried volume of the ligand present within 3 Å of the cobalt atom in the dormant species of the complex in which the ligand is coordinated to the cobalt atom, a₀, a₁, and a₂ are coefficients.)

The physical property values xᵢ are physical property values determined by performing first principles calculations on the dormant species, and the coefficients aᵢ are rational numbers up to two decimal places. The physical property values xᵢ may be determined by performing first principles calculations on the geometry-optimized dormant species.

As described above, x₁ in the equation (1a) is the NBO charge of the cobalt atom in the dormant species of the complex in which the ligand is coordinated to the cobalt atom. In the case where the initial structure of the dormant species in which the ligand is coordinated to the cobalt (II) of the Werner-type complex includes the ligand represented by the general formula (I), (III), or (IV) that is a monodentate ligand as in the chemical formula (A), x₁ of the equation (1a) is the NBO charge of the cobalt atom indicated by an arrow in the following formula (α1).

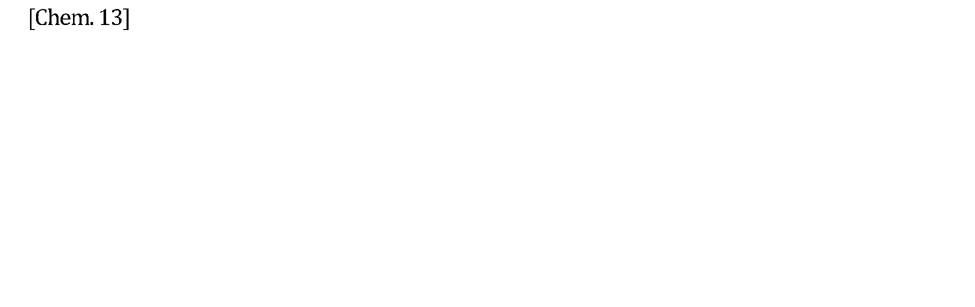

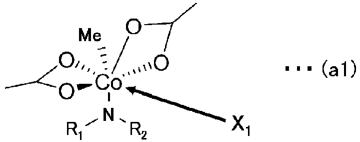

In the case where the initial structure of the dormant species in which the ligand is coordinated to the cobalt (II) of the Werner-type complex includes the ligand represented by the general formula (II) that is a bidentate ligand as in the above chemical formula (B), x₁ of the equation (1a) is the NBO charge of the cobalt atom indicated by the arrow in the following formula (α2).

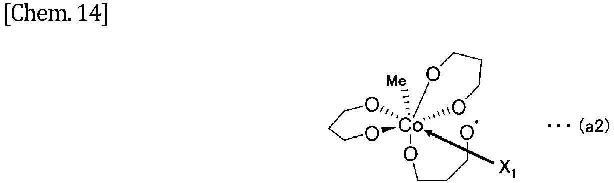

As described above, x₂ of the equation (1a) is the percent buried volume of the ligand within 3 Å of the cobalt atom in the dormant species of the complex in which the ligand is coordinated to the cobalt atom.

In the case where the initial structure of the dormant species in which the ligand is coordinated to the cobalt (II) of the Werner-type complex includes the ligand represented by the general formula (I), (III), or (IV) that is a monodentate ligand as in the chemical formula (A), x₂ of the equation (1a) is the percent buried volume of the ligand inside a sphere having a radius of 3 Å with the cobalt atom as a center, as illustrated in the following formula (β1).

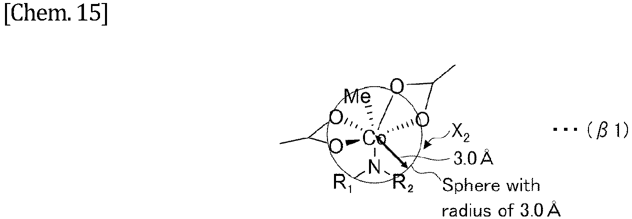

In the case where the initial structure of the dormant species in which the ligand is coordinated to the cobalt (II) of the Werner-type complex includes three molecules of the ligand represented by the general formula (II) that is a bidentate ligand as in the chemical formula (B), x₂ of the equation (1a) is the percent buried volume of the ligand inside a sphere having a radius of 3 Å with the cobalt atom as a center, as illustrated in the following formula (β2).

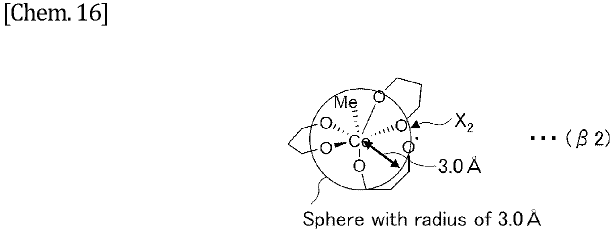

The physical property value acquisition unit 110 calculates the measured curing time T for the dormant species of the complex in which the ligand is coordinated, and a logarithmic value (log₁₀ T) of the measured curing time T.

The measured curing time T may be acquired from the material database 300, or may be actually measured. The measurement of the measured curing time T may be performed, for example, according to JIS K 6901 ("Room temperature curing properties (exothermic method)" of "Test methods for liquid unsaturated polyester resins").

The physical property value acquisition unit 110 transmits the acquired physical property values xᵢ and the log₁₀ T to the regression model generation unit 120.

### (Regression model generation unit )

The regression model generation unit 120 executes multiple regression analysis on the acquired physical property values xᵢ and log₁₀ T, thereby estimating coefficients a₀, a₁, and a₂ of the linear regression equation (1a) as the regression model. Specifically, the regression model generation unit 120 substitutes the physical property values xᵢ acquired for the ligand Lₙ (n is an integer) into the linear regression equation of the estimation target, thereby acquiring [T]_Lₙ. Specifically, [T]_Lₙ is an equation corresponding to the ligand Lₙ, and the number of [T]_Lₙ is n, which is the same as the total number of the ligands.

The regression model generation unit 120 determines a coefficient aᵢ (i = 0 to 2) to reproduce the measured curing time T for each of [T]_Lₙ. For example, reproducibility of the log₁₀ T may be evaluated by a determination coefficient R² that is a square of a correlation coefficient. It is known that as the determination coefficient R² approaches 1, a stronger correlation between two values is observed. In general, a meaningful correlation can be confirmed when the determination coefficient R² is 0.6 or greater. The regression model generation unit 120 may determine that sufficient reproducibility is achieved, for example, when R² is 0.6 or greater.

The regression model generation unit 120 may determine the coefficients aᵢ (i = 0 to 2) using, for example an evolutionary algorithm so that the determination coefficient R² of the log₁₀ T and the log₁₀ T becomes closer to 1.

For example, the estimation of the regression coefficients using the evolutionary algorithm can be implemented by using the evolutionary algorithm in a solver included in Excel (registered trademark).

As calculation conditions, for example, the convergence, the mutation rate, the population size, the random seed, and the maximum time with no improvement may be set to predetermined values as appropriate, and the regression coefficients may be estimated by the solver. In addition, the upper limit and lower limit of the variables are not necessary, and the values of aᵢ output as calculation results may not be limited to positive or negative.

The regression model generation unit 120 can derive a linear regression equation including the predetermined coefficients aᵢ (i = 0 to 2) from the physical property values xᵢ obtained for the ligand Lₙ, which include the NBO charge x₁ of the cobalt atom in the dormant species of the complex in which the ligand is coordinated to the cobalt atom, and the percent buried volume x₂ of the ligand present within 3 Å of the cobalt atom in the dormant species of the complex in which the ligand is coordinated to the cobalt atom.

The regression model generation unit 120 determines log₁₀ [T] from the physical property values xᵢ of the dormant species of the complex in which the ligand is coordinated to the cobalt organic acid salt, using the generated linear regression equation.

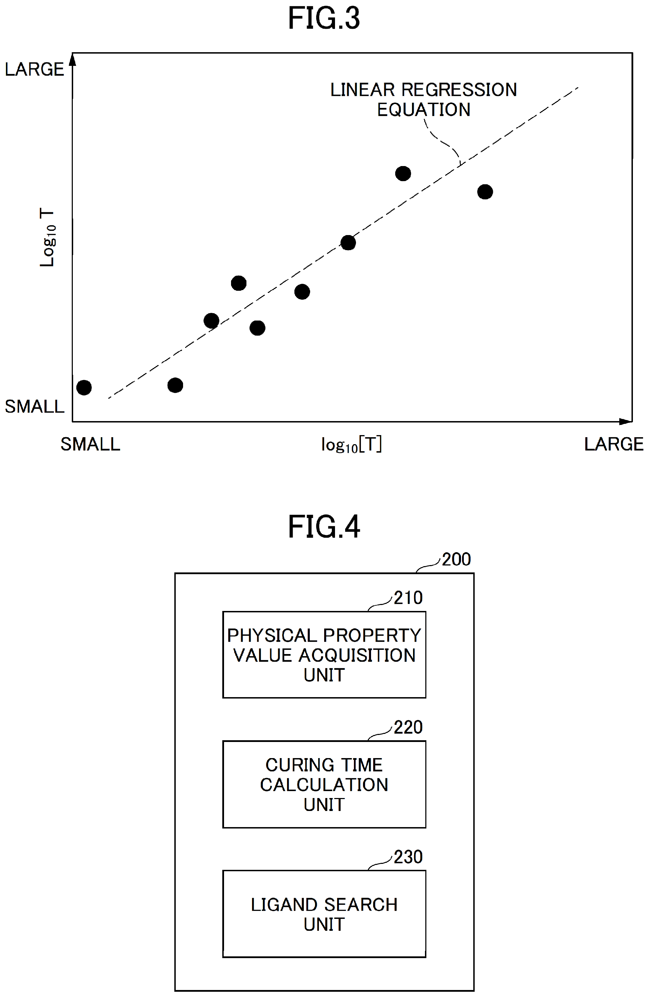

One example of the relationship between log₁₀ [T] and log₁₀ T is illustrated in Fig. 3. In Fig. 3, the constant multiple of log₁₀ [T] may be performed, or the intercept may be horizontally shifted to match with the log₁₀ T. As illustrated in Fig. 3, a linear regression equation is determined by adjusting the slope of the linear approximation equation representing the relationship between log₁₀ [T] and log₁₀ T to be 1.0. When the determination coefficient R² of the graph of Fig. 3 is a predetermined value (e.g., 0.6), log₁₀ [T] can be predicted with sufficient correlation. The operation of shifting the log₁₀ [T] in Fig. 3 is not essential as long as the log₁₀ [T] is adjusted to fit the scale of the log₁₀ T

The regression model generation unit 120 transmits the regression model, which is represented by the estimated linear regression equation using the equation (1a) as described above, to the ligand search device 200.

### <Ligand search device>

A configuration of the ligand search device 200 will be described. The ligand search device 200 predicts log₁₀ [T] for a ligand of a curing accelerator represented by any of the general formulae (I) to (VI), where the log₁₀ [T] is calculated by the linear regression equation of the equation (1) using the regression model acquired by the regression model generation device 100, and searches for a ligand of the curing accelerator with which the log₁₀ [T] is equal to or lower than a predetermined value.

Fig. 4 is a block diagram illustrating a functional configuration of the ligand search device 200. As illustrated in Fig. 4, the ligand search device 200 includes a physical property value acquisition unit 210, a curing time calculation unit 220, and a ligand search unit 230.

The physical property value acquisition unit 210 acquires physical property values for each of ligands of a curing accelerator. Specifically, similarly to the physical property value acquisition process described in association with the regression model generation device 100, the physical property value acquisition unit 210 performs geometry optimization on the material data of a processing target material acquired from the material database 300, and performs first principles calculations on the geometry-optimized dormant species to determine the above-described physical property values xᵢ, which include the NBO charge x₁ of the cobalt atom in the dormant species of the complex in which the ligand is coordinated to the cobalt atom and the percent buried volume x₂ of the ligand present within 3 Å of the cobalt atom in the dormant species of the complex in which the ligand is coordinated to the cobalt atom.

The curing time calculation unit 220 substitutes the acquired physical property values xᵢ into the linear regression equation to calculate log₁₀ [T] of the predicted curing time [T], and provides the calculated log₁₀ [T] to the ligand search unit 230.

The ligand search unit 230 determines whether the calculated log₁₀ [T] is equal to or lower than the predetermined value, and determines the curing accelerator including the ligand with which log₁₀ [T] is equal to or lower the predetermined value as a search target curing accelerator.

As the log₁₀ [T] calculated by the linear regression equation of the equation (1) becomes small, the measured curing time T of the curing accelerator including the cobalt organic acid salt and the ligand is shortened. Therefore, when the log₁₀ [T] is equal to or lower than the predetermined value, it is determined that the curing time of the curing accelerator including such a ligand is short.

The predetermined value may be, for example, 5.0. In the case where the log₁₀ [T] exceeds the predetermined value, the ligand search unit 230 determines that the curing accelerator including such a ligand is not a search target curing accelerator, and begins searching of a subsequent ligand of a curing accelerator.

### (Hardware configuration)

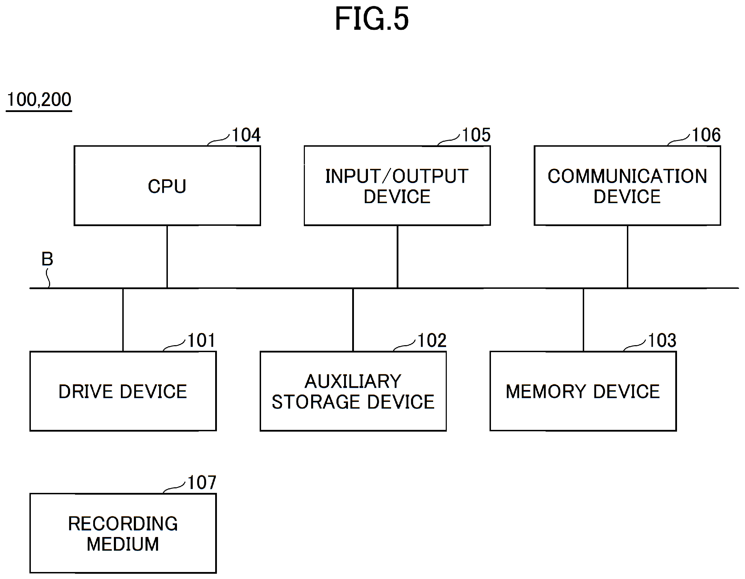

The regression model generation device 100 and the ligand search device 200 may have, for example, a hardware configuration as illustrated in Fig. 5. Specifically, the regression model generation device 100 and the ligand search device 200 include a drive device 101, an auxiliary storage device 102, a memory device 103, a central processing unit (CPU) 104, an input/output device 105, and a communication device 106, which are connected to one another via a bus B.

Various computer programs including programs for implementing various functions and processes in the regression model generation device 100 and the ligand search device 200, which will be described later, may be provided by a recording medium 107, such as a compact disk-related only memory (CD-ROM) or the like. As the recording medium 107 storing a program is set in the drive device 101, the program is installed from the recording medium 107 into auxiliary storage device 102 via the drive device 101. However, the program is not necessarily installed using the recording medium 107, and the program may be downloaded from any external device via a network or the like. The auxiliary storage device 102 stores the installed program, and also stores necessary files, data, and the like. When an instruction to start the program is given, the memory device 103 reads out the program or data from the auxiliary storage device 102 and stores the program or data. The CPU 104 functioning as a processor executes various functions and processes of the regression model generation device 100 and the ligand search device 200, as described below, according to various data, such as the program or parameters necessary to execute the program, and the like, which are stored in the memory device 103. The input/output device 105 provides an interface between the regression model generation device 100, the ligand search device 200, and user, and may be configured by, for example, an input device, such as a keyboard, a mouse, a touch screen, or the like, and an output device, such as a display, a speaker, or the like. The communication device 106 executes various communication processes for communicating with an external device.

However, the regression model generation device 100 and the ligand search device 200 are not limited to have the above-described hardware configuration, and may be implemented by any other appropriate hardware configurations or a configuration via a network.

### <Ligand search method>

The ligand search method according to the present embodiment will be described. In the ligand search method according to the present embodiment, the ligand search system 1 having the configuration as illustrated in Fig. 1 acquires material data (e.g., a curing time) associated with the curing accelerator from the material database 300, and generates a regression model for predicting a curing time from physical property values of the curing accelerator, and the ligand search device 200 searches for a ligand of a curing accelerator using the generated regression model. The ligand search method according to the present embodiment is, for example, executed by the above-described ligand search system 1. Specifically, the ligand search method may be executed by a processor of the regression model generation device 100 or the ligand search device 200 constituting the ligand search system 1. Since the method for searching the material according to the present embodiment can be performed by the above-described ligand search system 1, part of the features of each step, which has been already described for the above-described ligand search system 1, will be omitted.

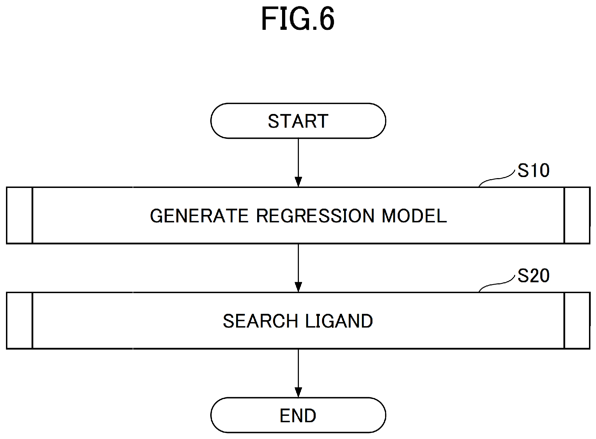

Fig. 6 is a flowchart explaining the ligand search method according to the present embodiment. As illustrated in Fig. 6, the ligand search method according to the present embodiment includes a regression model generation process S10 and a ligand search process S20.

### [Regression model generation process]

The regression model generation process S10 will be described. The regression model generation process is executed, for example, by the above-described regression model generation device 100. Specifically, the regression model generation process may be executed by a processor of the regression model generation device 100.

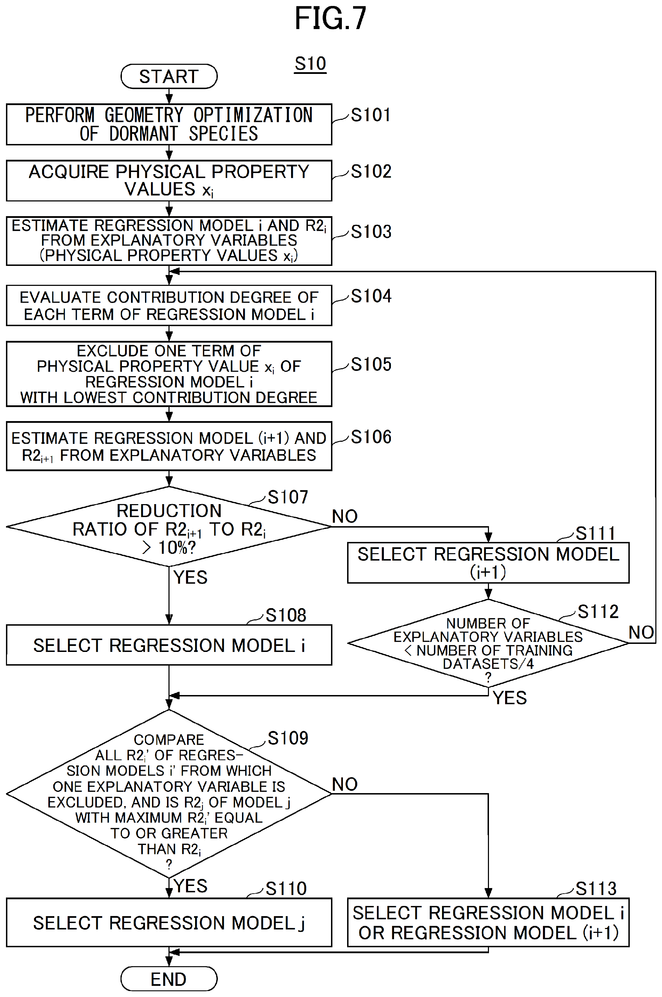

Fig. 7 is a flowchart illustrating one example of the regression model generation process S10. As illustrated in Fig. 7, in the regression model generation process S10, the regression model generation device 100 executes geometry optimization of a dormant species on the material data acquired from the material database 300 (geometry optimization step: step S101).

Next, the regression model generation device 100 acquires physical property values xᵢ from the geometry-optimized dormant species, which include an NBO charge x₁ of a cobalt atom in the dormant species of the complex in which a ligand is coordinated to the cobalt atom, and percent buried volume x₂ of the ligand within 3 Å of the cobalt atom in the dormant species of the complex in which the ligand is coordinated to the cobalt atom (physical property value acquisition step: step S102).

Next, the regression model generation device 100 executes multiple regression analysis using the acquired physical property values xᵢ as explanatory variables to estimate a linear regression equation as a regression model i (i is an integer of 1 to 8), thereby estimating a determination coefficient R2ᵢ of the linear regression equation (first regression model and determination coefficient estimation step: step S103).

For example, the regression model generation device 100 substitutes the physical property values xᵢ into the following equation (10) that is a linear regression equation of an estimation target, thereby estimating a regression model i.
[Math 1] ${log}_{10} \left[T\right] = {a}_{0} + {\sum }_{i = 1}^{8} {a}_{i} ⋅ {x}_{i}$ (10)

When i of the equation (10) is 1 to 8, the physical property values xᵢ are as follows.
x₁: SOMO
x₂: LUMO
x₃: distance between Me and Co
x₄: NBO charge of Co
x₅: NBO charge of carbon of Me
x₆: average value of NBO charges of double-bonded oxygens
x₇: average distance between Co and oxygen atoms
x₈: percent buried volume of ligand in 3 Å sphere with Co as center

The regression model generation device 100 determines each of coefficients aᵢ of the linear regression equation to reproduce the measured curing time T, and estimates a regression model i based on the determination coefficient R².

When the determination coefficient R² is 0.6 or greater, the regression model generation device 100 estimates that there is reproducibility.

The regression model generation device 100 determines coefficients aᵢ using an evolutionary algorithm, for example, so that the determination coefficient R² of log₁₀ [T] and the log₁₀ T becomes closer to 1. In the case where the multiple regression analysis is executed using the evolutionary algorithm in the solver included in Excel (registered trademark), regression coefficients may be estimated by the solver, for example, by appropriately setting, for example, the convergence, the mutation rate, the population size, the random seed, and the maximum time with no improvement, to predetermined values, respectively, as calculation conditions.

Next, the regression model generation device 100 evaluates a contribution degree of each of terms of the linear regression equation that is the regression model i (evaluation step of contribution degree of each term of regression model: step S104).

The contribution degree of each of terms of the regression mode can be evaluated by executing the following two equations (11) and (12), and selecting the higher contribution degree among the contribution degrees obtained by the equations (11) and (12). The training data of the equations (11) and (12) is the physical property values xi.$Contribution degree = \left|coefficient {a}_{i}\times average value of training data\right|$ (11) $Contribution degree = \left|coefficient {a}_{i }\times standard deviation of training data\right|$ (12)

Next, the regression model generation device 100 excludes one term of the physical property value xᵢ with which the contribution degree is lowest among the evaluated contribution degrees of all the terms of the physical property values xᵢ of the linear regression equation represented by the equation (10) that is the regression model i (exclusion step: step S105).

Next, similarly to the above-described first regression model and determination coefficient estimation step (step S103), the regression model generation device 100 executes a multiple regression analysis using the acquired physical property values xᵢ as explanatory variables to estimate a linear regression equation as a regression model (i + 1), thereby estimating a determination coefficient R2ᵢ₊₁ of the linear regression equation (second regression model and determination coefficient estimation step: step S106).

The estimation methods of the linear regression equation as the regression model (i + 1) and the determination coefficient R2ᵢ₊₁ of the linear regression equation are the same as in the first regression model and determination coefficient estimation step (step S103), and therefore the details of the estimation methods will be omitted.

Next, the regression model generation device 100 compares the determination coefficient R2ᵢ estimated in the first regression model and determination coefficient estimation step (step S103) and the determination coefficient R2ᵢ₊₁ estimated in the second regression model and determination coefficient estimation step (step S106), and determines whether a reduction ratio of the determination coefficient R2ᵢ₊₁ to the determination coefficient R2ᵢ exceeds 10% (first determination step: step S107).

The reduction ratio of the determination coefficient R2ᵢ₊₁ to the determination coefficient R2ᵢ is represented by the following equation (13). $\left(\left({R 2}_{i + 1}-{R 2}_{i}\right)/{R 2}_{i}\right) \times 100 < - 10 \left[%\right]$ (13)

In the case where the regression model generation device 100 determines that the reduction ratio ((R2ᵢ₊₁/R2ᵢ) × 100) of the determination coefficient R2ᵢ₊₁ to the determination coefficient R2ᵢ exceeds 10% (step S107: YES), it is determined that the regression model i is more accurate than the regression model (i+1), thereby selecting (picking) the regression model i (first selection step: step S108).

On the other hand, in the case where the regression model generation device 100 determines the reduction ratio of the determination coefficient R2ᵢ₊₁ to the determination coefficient R2ᵢ is equal to or less than 10% (step S107: NO) in the first determination step (step S107), it is determined that the regression model (i + 1) is more accurate than the regression model i, thereby selecting the (i + 1) (third selection step: step S111).

Then, the regression model generation device 100 determines whether the number of the physical property values xᵢ as explanatory variables is less than 1/4 of the number of training datasets (the number of training datasets/4) (third determination step: step S112).

The number of the explanatory variables is not limited to less than 1/4 of the number of the training datasets, as long as the number of the explanatory variables is sufficiently smaller than the number of the training datasets and functions as an index for avoiding overfitting. The number of the explanatory variables may be less than any number of training datasets as appropriate, and may be, for example, less than 1/2.

In the case where the regression model generation device 100 determines that the number of the explanatory variables is less than 1/4 of the number of the training datasets (step S112: YES), it is determined that the explanatory variables have been able to avoid overfitting with respect to the number of the training datasets, and the process proceeds to a second determination step (step S109).

On the other hand, in the case where the regression model generation device 100 determines that the number of the explanatory variables is equal to or greater than 1/4 of the number of the training datasets (step S112: NO), it is determined that the explanatory variables have not been able to avoid overfitting with respect to the number of the training datasets, and the process proceeds to the first regression model and determination coefficient estimation step (step S103).

In this manner, the regression model generation device 100 repeats the processes of the first regression model and determination coefficient estimation step (step S103) to the first selection step (step S108), the third selection step (step S111), and the third determination step (step S112) to reduce, one by one, the coefficients aᵢ as arbitrary variables of the linear regression equation represented by the equation (10) as the regression model i, and performs the repetition of the processes until lastly two coefficients aᵢ remain. Specifically, the regression model i obtained by the initial estimation has 8 physical property values xᵢ (x₁ to x₈), and the number of the physical property values xᵢ is reduced one by one until ultimately two terms (i = 1 and 2) of the physical property values xᵢ remain. As a result, the following equation (1) is obtained. ${log}_{10} \left[T\right] = {a}_{0} + {a}_{1} {x}_{1} + {a}_{2} {x}_{2}$ (1)

Note that x₁ and x₂ of the equation (1) are different from x₁ and x₂ of the equation (10), and means lastly remaining two physical property values xᵢ among the eight physical property values xᵢ. In the equation (1), a₁ and a₂ are also different from a₁ and a₂ of the equation (10), and may be appropriately determined according to the lastly remaining two physical property values xᵢ.

For example, the regression model generation device 100 determines coefficients a₁ and a₂ in the equation (1) using an evolutionary algorithm, so that the determination coefficient R² of the log₁₀ [T] and the log₁₀ T becomes closer to 1. In the case where the multiple regression analysis is executed using the evolutionary algorithm of the solver included in Excel (registered trademark), estimation of regression coefficients may be performed by the solver by appropriately setting, for example, the convergence, the mutation rate, the population size, the random seed, and the maximum time with no improvement, to predetermined values as appropriate, as calculation conditions. The regression model generation device 100 determines the coefficient a₀ so that the absolute values of the log₁₀ [T] and the log₁₀ T are matched.

Next, the regression model generation device 100 estimates all regression models i' from which one physical property value xᵢ as an explanatory variable is excluded, and estimates and compares determination coefficients R2i' for all the linear regression equations as the regression models i'to determine the regression model j with which the determination coefficient R2i' is maximum among all the regression models i', and determines whether the determination coefficient R2ⱼ of the regression model j is equal to or higher than the determination coefficient R2ᵢ of the regression model i (second determination step: step S109).

The regression models i' from which one physical property value xᵢ is excluded refers to linear regression equations in which at least one of terms of the linear regression equation of the equation (10) as the regression model i is excluded. Specifically, in the case where the linear regression equation is a₀ + a₁x₁ + a₂x₂ + a₃x₃ + a₄x₄ + a₅x₅ + a₆x₆ + a₇x₇ + a₈x₈, the regression models i' refer to 8 patterns of the linear regression equations from which a₁x₁, a₂x₂, a₃x₃, a₄x₄, a₅x₅, a₆x₆, a₇x₇, or a₈x₈ is excluded, respectively.

The estimation of the determination coefficients R2ᵢ' of all the linear regression equations means estimation of the determination coefficients R2ᵢ' of all of the linear regression equations in which any one of terms of the linear regression equation of the equation (10) is excluded. Specifically, in the case where the linear regression equation is a₀ + a₁x₁ + a₂x₂ + a₃x₃ + a₄x₄ + a₅x₅ + a₆x₆ + a₇x₇ + a₈x₈, determination coefficients R2₁ to R2₈ of 8 patterns of the linear regression equations from which a₁x₁, a₂x₂, a₃x₃, a₄x₄, a₅x₅, a₆x₆, a₇x₇, or a₈x₈ is excluded, respectively, are estimated.

In the evaluation step of the contribution degree of each term of the regression model (step S104),it is difficult to compare between the terms of the linear regression equation only by the contribution degree, as the comparison in the contribution degree between the terms shows a very slight difference. By estimating determination coefficients R2ᵢ' of all the linear regression equations serving as the regression models i' in which one of the physical property values xᵢ is excluded and comparing with the determination coefficients R2ⱼ of the regression model j, which term of the linear regression equation is preferable to remain can easily be compared and determined.

In the case where the regression model generation device 100 determines that the determination coefficient R2ⱼ is equal to or greater than the determination coefficient R2ᵢ (step S109: YES), it is determined that the regression model j is more accurate than the regression model i or the regression model (i + 1), and the regression model j is selected (second selection step: step S110).

In addition, in the case where the regression model generation device 100 determines that the determination coefficient R2ᵢ is less than the determination coefficient R2ᵢ (step S109: NO) in the second determination step (step S109), it is determined that the regression model i is more accurate than the regression model j or the regression model (i + 1), and the regression model i is selected (fourth selection step: step S113).

As described above, after selecting the regression model i, the regression model (i + 1), or the regression model j, the regression model generation device 100 ends the regression model generation process, and provides the lastly acquired linear regression equation to the ligand search device 200 as the generated regression model.

### [Ligand search process]

The ligand search process S20 will be described. The ligand search process S20 is executed by the above-described ligand search device 200. For example, the ligand search process S20 may be executed by a processor of the ligand search device 200.

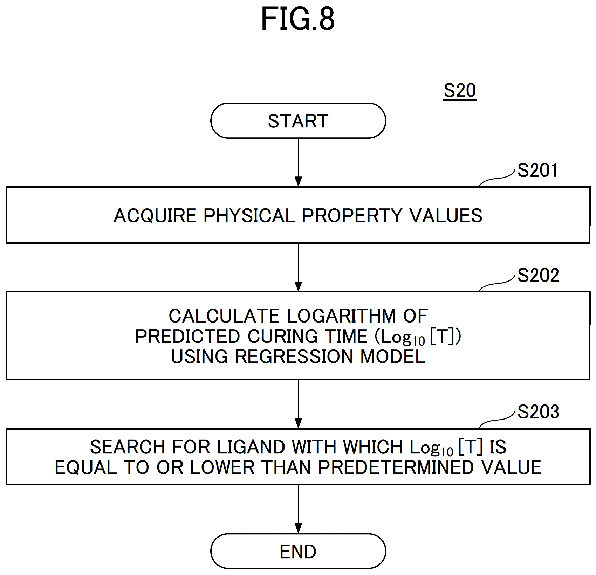

Fig. 8 is a flowchart illustrating one example of the ligand search process S20. As illustrated in Fig. 8, in the ligand search process S20, the ligand search device 200 acquires physical property values xᵢ associated with curing accelerators including ligands of a search target (physical property value acquisition step: step S201).

Next, the ligand search device 200 calculates log₁₀ [T] from the acquired physical property values xᵢ using the regression model (logarithmic value of predicted curing time calculation step: step S202).

Next, the ligand search device 200 determines whether the calculated log₁₀ [T] is equal to or lower than the predetermined value (e.g., 5.0), and searches for a curing accelerator including a ligand with which the log₁₀ [T] is equal to or lower than the predetermined value (searching step: step S203).

When the curing accelerator including the ligand with which the calculated log₁₀ [T] is equal to or lower than (e.g., 5.0) is detected, the ligand search device 200 determines and outputs the detected curing accelerator as a search target curing accelerator.

In the ligand search method according to the present embodiment, a ligand is searched using a value of the log₁₀ [T] determined from the regression model as a reference, and therefore it is possible to search a ligand of a curing accelerator with which a curing time can be shortened when a radically polymerizable resin is cured at room temperature. Thus, the ligand search method according to the present embodiment can be used for selection of a curing accelerator capable of shortening a curing time when a radically polymerizable resin is cured at room temperature.

As described above, the ligand search system 1 includes the regression model generation device 100 and the ligand search device 200, where the regression model generation device 100 generates a regression model for predicting a logarithmic value of a curing time from physical property values of a curing accelerator, and the ligand search device 200 can search for a ligand of the curing accelerator with reference to log₁₀ [T] predicted from the physical property values xᵢ of the curing accelerators including various ligands using the generated regression model. Therefore, the ligand search system 1 can search for a ligand of a curing accelerator with which a curing time can be shortened when a radically polymerizable resin is cured at room temperature.

Since the ligand search system 1 can search for a ligand of a curing accelerator with high accuracy, a curing accelerator effective for curing a radically polymerizable resin at room temperature in a short time can be selected.

### <Curing accelerator>

The curing accelerator searched by the ligand search method according to the present embodiment will be described. The curing accelerator includes a cobalt organic acid salt and a ligand, which can accelerate curing of a radically polymerizable resin.

Examples of the radically polymerizable resin include unsaturated polyester resins, vinyl ester resins, and the like.

Examples of the cobalt organic acid salt include cobalt octylate, cobalt naphthenate, cobalt stearate, cobalt neodecanoate, and the like.

The ligand includes an amine structure or a β-diketone structure. Examples of the ligand include ligands represented by the general formulae (i) to (xiv) and the like.

The ligand may be synthesized by a generally known synthesis method. Examples of the generally known synthesis method include Japanese Unexamined Patent Application Publication No. 2004-26765, the specification of German Patent No. 1089760, Japanese Translation of PCT International Application Publication No. 2013-522343, International Publication No. WO 95/21525, Calter et al., Journal of Organic Chemistry 2004, vol. 69 (4), p 1270-1275, Hilgenkamp et al., Tetrahedron 2001, vol. 57, #42, p 8793-8800, and the like.

The physical property values of the curing accelerator includes an average distance between oxygen atoms and a cobalt atom in a dormant species of a complex in which a ligand is coordinated to the cobalt atom, and percent buried volume of the ligand present within 3 Å of the cobalt atom in the dormant species of the complex in which the ligand is coordinated to the cobalt atom.

The curing time is, for example, a time required for the radically polymerizable resin to be cured at room temperature (e.g., 25°C) using ketone peroxide as a curing agent, and the above curing accelerator.

In the case where the radically polymerizable resin is, for example, an unsaturated polyester resin, a curing time of the unsaturated polyester resin can be measured, for example, according to JIS K 6901("Room temperature curing properties (exothermic method)" of "Test methods for liquid unsaturated polyester resins").

The liquid unsaturated polyester resin is a solution in which the unsaturated polyester resin is dissolved in a monomer (e.g., styrene) capable of polymerizing the unsaturated polyester resin.

Although the embodiments have been described above, the embodiments are merely presented as examples, and the present invention is not limited by the embodiments. The above embodiments can be implemented in various other forms, and various combinations, omissions, substitutions, changes, and the like can be made without departing from the gist of the invention. These embodiments and modifications thereof are included in the scope and gist of the invention, and are included in the invention described in the claims and the scope of equivalents of the claims.

### Examples

Hereinafter, the present embodiment will be more concretely described through Examples, but the present embodiment is not limited by Examples.

### <Reference Examples 1 to 13>

### [Preparation of ligand search system]

A ligand search system 1 including a regression model generation device 100 and a ligand search device 200, as illustrated in Fig. 1, was prepared.

### [Generation of regression model]

### (Preparation of ligands)

As material data used for generation of a regression model, the following ligands L₁ to L₁₃ of Reference Examples 1 to 13 were acquired.

### ((Ligands of Reference Examples 1 to 13))

### Ligand (L₁) of Reference Example 1:

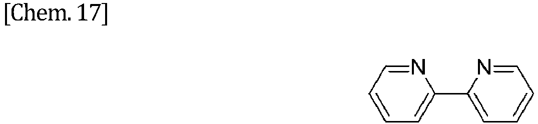

### Ligand (L₂) of Reference Example 2:

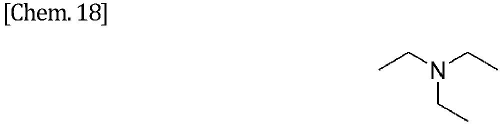

### Ligand (L₃) of Reference Example 3:

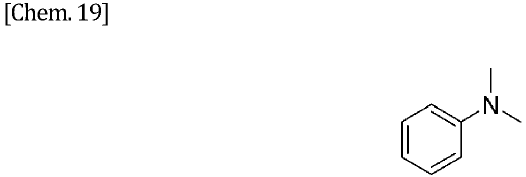

### Ligand (L₄) of Reference Example 4:

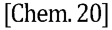

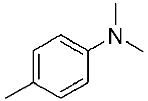

### Ligand (L₅) of Reference Example 5:

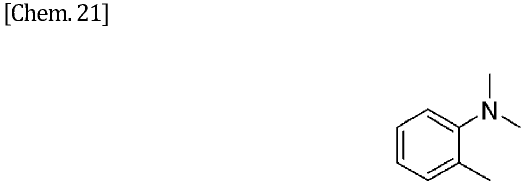

### Ligand (L₆) of Reference Example 6:

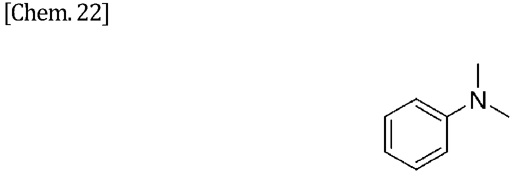

### Ligand (L₇) of Reference Example 7:

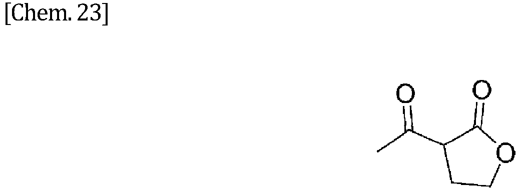

### Ligand (L₈) of Reference Example 8:

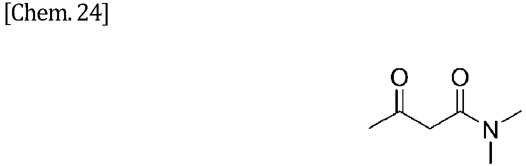

### Ligand (L₉) of Reference Example 9:

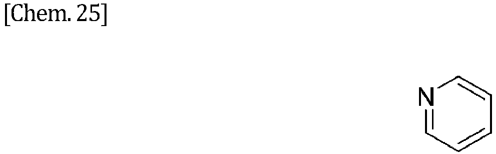

### Ligand (L₁₀) of Reference Example 10:

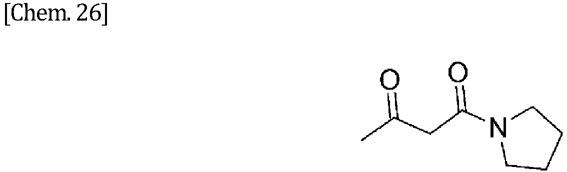

### Ligand (L₁₁) of Reference Example 11:

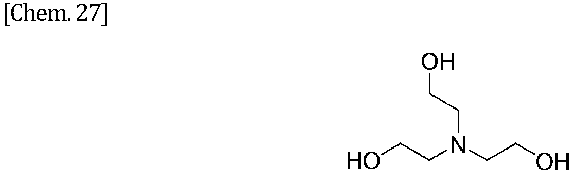

### Ligand (L₁₂) of Reference Example 12:

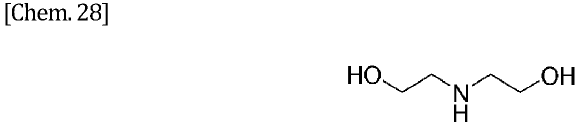

### Ligand (L₁₃) of Reference Example 13:

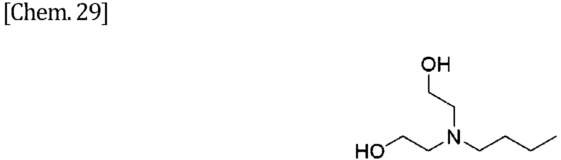

The ligands (L₁ to L₁₃) of Reference Examples 1 to 13 are specific examples of the ligands represented by any of the general formulae (I) to (IV).
Ligands of Reference Examples 2 and 11 to 13: specific examples of the ligand represented by the general formula (III)
Ligands of Reference Examples 3 to 6: specific examples of the ligand represented by the general formula (I)
Ligands of Reference Examples 7, 8, and 10: specific examples of the ligand represented by the general formula (II)
Ligands of Reference Examples 1 and 9: specific examples of the ligand represented by the general formula (IV)

### (Production of dormant species)

Dormant species in which ligands L₁ to L₁₃ of Reference Examples 1 to 13 were coordinated, respectively, were geometry optimized by the regression model generation device 100, thereby producing the geometry-optimized dormant species in which the ligands L₁ to L₁₃ of Reference Examples 1 to 13 were coordinated, respectively.

### (Calculation of physical property values)

The regression model generation device 100 performed quantum chemical calculations on the geometry-optimized dormant species, in which the ligands L₁ to L₁₃ of Reference Examples 1 to 13 were coordinated, respectively, using quantum chemical calculation software (Gaussian 09 of Gaussian, Inc.), thereby calculating the following physical property values xᵢ. Gaussian 09 used RωB97XD as a functional and 6 - 31⁺G(d) as a basis function based on the unrestricted density-functional theory. The frequency analysis was performed in all calculations to confirm that the number of the imaginary vibration frequencies was zero, thereby determining that the calculated structures were converged at the bottom of the potential energy curve.

### ((Physical property values xᵢ))

Physical property value x₁: an NBO charge of a cobalt atom in a dormant species of a complex in which the ligand is coordinated to the cobalt atom
Physical property value x₂: percent buried volume of the ligand within 3 Å of the cobalt atom in which the dormant species of the complex in which the ligand is coordinated to the cobalt atom

### (Calculation of measured curing time T and logarithmic value of measured curing time T)

A measured curing time T and logarithmic value of the measured curing time T (log₁₀ T) of each of the dormant species of the complexes in which ligands of Reference Examples 1 to 13 were coordinated, respectively, were calculated. The measured curing time T was acquired from the above material database.

### (Estimation of coefficients of linear regression equation (1a))

The regression model generation unit 120 executed a multiple regression analysis on the physical property values xᵢ and log₁₀ T acquired by the physical property value acquisition unit 110, and estimated coefficients a₀, a₁, and a₂ of a linear regression equation (1a) as a regression model. Specifically, the physical property values xᵢ acquired for each of the ligands Lⱼ (j = 1 to 13) of Reference Examples 1 to 13 were substituted into the linear regression equation of an estimation target, thereby acquiring [T]_Lⱼ (j = 1 to 13) that was the equation corresponding to the ligand (Lⱼ). The coefficients ai (i = 0 to 2) of [T]_Lⱼ were each determined to reproduce the measured curing time T of each [T]_Lⱼ. The reproducibility of the log₁₀ T was evaluated by a determination coefficient R² that was a square of a correlation coefficient. It is considered that the closer the determination coefficient R² is to 1, the stronger the correlation of the two values is. When R² was 0.6 or greater, it was evaluated that there was reproducibility.

The coefficients aᵢ (i = 1 and 2) of the linear regression equation (1a) were determined using an evolutionary algorithm so that the determination coefficient R² of the log₁₀ T and the log₁₀ [T] became closer to 1. For the determination of the coefficients aᵢ (i = 1 and 2), the reduction of the coefficients to the coefficients a₁ and a₂, which were arbitrary variables, was performed by reducing the coefficients one by one. The coefficient a₀ was determined so that an absolute value of the log₁₀ [T] and an absolute value of the log₁₀ T were matched.

The estimation of the regression coefficients using the evolutionary algorithm was performed using the evolutionary algorithm in the solver included in Excel (registered trademark). The estimation of the regression coefficients by the solver was performed by setting, as conditions of calculations, the convergence to 0.0001, the mutation rate to 0.075, the population size to 100, the random seed to 100, and the maximum time with no improvement to 300.

### (Derivation of linear regression equation (2a))

A linear regression equation was derived from the physical property values xᵢ acquired for each of the ligands Lᵢ of Reference Examples 1 to 13, where the physical property values xᵢ included the NBO charge x₁ of the cobalt atom in the dormant species of the complex in which the ligand was coordinated to the cobalt atom, and the percent buried volume x₂ of the ligand present within 3 Å of the cobalt atom in the dormant species of the complex in which the ligand was coordinated to the cobalt atom.

The formula (2a) represented by the following linear regression equation was derived from the physical property values xᵢ acquired for each of the ligands Lᵢ of Reference Examples 1 to 13, where the physical property values xᵢ included the NBO charge x₁ of the cobalt atom in the dormant species of the complex in which the ligand was coordinated to the cobalt atom, and the percent buried volume x₂ of the ligand present within 3 Å of the cobalt atom in the dormant species of the complex in which the ligand was coordinated to the cobalt atom. ${log}_{10} \left[T\right] \left({x}_{i}\right) = 5.20950 {x}_{1} - 0.50360 {x}_{2} + 47.001$ (2a)

### (Calculation of logarithmic value of predicted curing time [T] (log₁₀ [T]))

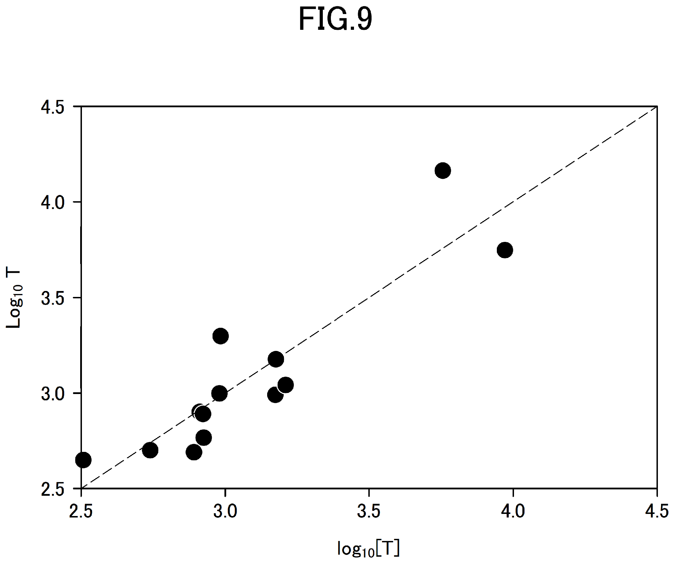

Using the derived equation (2a), log₁₀ [T] was calculated from the physical property values xᵢ of the dormant species of the complex in which each of the ligands of Reference Examples 1 to 13 was coordinated to the cobalt organic acid salt. The relationship between the log₁₀ T and the log₁₀ [T] is depicted in Fig. 9. In Fig. 9, a constant multiple of log₁₀ [T] was performed, and the intercept was horizontally shifted to match with the log₁₀ T of each of Reference Examples 1 to 13, so that the slope of the linear approximation formula representing the relationship between the log₁₀ T and the log₁₀ [T] was adjusted to 1.0. Since the determination coefficient R² of the linear approximation formula of the equation (2a) was approximately 0.86 as illustrated in Fig. 9, the linear approximation formula of the equation (2a) predicted the log₁₀ [T] with sufficient correlation with the log₁₀ T

The physical property values x₁ and x₂, log₁₀ [T], measured curing time T, and log₁₀ T of the dormant species of the complex in which each of the ligands of Reference Examples 1 to 13 was coordinated are presented in Table 1.

**[Table 1]**

| Ref. Ex. | Physical property values | | Logarithm of predicted curing time T (log₁₀[T]) | Measured curing time T [s] | Logarithm of measured curing time T (Log₁₀ T) |
|---|---|---|---|---|---|
| | X₁ | X₂ | | | |
| 1 | 0.725 | 93.2 | 3.84 | 14492 | 4.16 |
| 2 | 0.773 | 95.4 | 2.98 | 980 | 2.99 |
| 3 | 0.769 | 95.6 | 2.86 | 582 | 2.76 |
| 4 | 0.769 | 95.6 | 2.86 | 788 | 2.90 |
| 5 | 0.775 | 95.7 | 2.84 | 498 | 2.70 |
| 6 | 0.770 | 95.6 | 2.87 | 756 | 2.88 |
| 7 | 0.740 | 95.0 | 3.01 | 974 | 2.99 |
| 8 | 0.811 | 96.4 | 2.68 | 502 | 2.70 |
| 9 | 0.701 | 92.6 | 4.02 | 5584 | 3.75 |
| 10 | 0.885 | 97.1 | 2.71 | 448 | 2.65 |
| 11 | 0.773 | 95.4 | 2.98 | 1956 | 3.29 |
| 12 | 0.779 | 94.9 | 3.07 | 1088 | 3.04 |
| 13 | 0.732 | 94.8 | 3.27 | 1480 | 3.17 |

### (Generation of regression model)

The linear regression equation represented by the above equation (2a) was used as the regression model.

### [Search for ligand]

Using the specific example of the ligand represented by the following general formula (II) (ligand of Evaluation Example h1) as test data, whether the ligand of Evaluation Example h1 was the ligand with which log₁₀ [T] calculated by the equation (2a) represented as the linear regression equation was 5.0 or less was confirmed by the ligand search device 200. Specifically, after determining physical property values xᵢ in the same manner as the ligands of Reference Examples 1 to 13, the physical property values xᵢ were substituted into the equation (2a), thereby obtaining log₁₀ [T].

### Ligand of Evaluation Example h1:

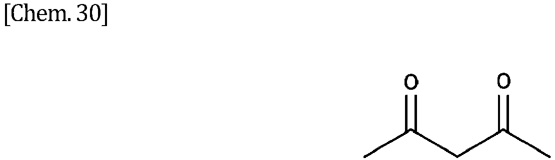

The ligand search device 200 caused the ligand search unit 230 to calculate log₁₀ [T] when the above ligand was used, and to obtain a search result based on the calculated value of the log₁₀ [T]. When the value of the log₁₀ [T] was 5.0 or less, the ligand was determined as being effective for shortening a curing time (determination result "A" in Table 2). When the value of the log₁₀ [T] was greater than 5.0, the ligand was determined as being not effective for shortening a curing time (determination result "B" in Table 2). One example of the calculated log₁₀ [T] and the search result is presented in Table 2.

**[Table 2]**

| | | Physical property values | | Logarithm of predicted curing time T (log₁₀[T]) | Judgment |
|---|---|---|---|---|---|
| | | X₁ | X₂ | | |
| Evaluation Example | h1 | 0.829 | 95.9 | 3.02 | A |

It was confirmed from Table 2 that the log₁₀ [T] could be 3.0 or less when a radically polymerizable resin was cured at room temperature with a redox initiator including ketone peroxide and a cobalt organic acid salt using the ligand of Evaluation Example h1.

The ligand of Evaluation Example h1 is a ligand represented by the general formula (II) among the general formulae (I) to (IV). Using the ligand of Evaluation Example h1, the log₁₀ [T] substantially corresponding to the linear regression equation represented by the equation (2a) was obtained. The linear regression equation represented by the equation (2a) is an equation obtained by the calculation based on the ligand represented by the general formula (II) and the ligands represented by the general formulae (I), (III), and (IV). Therefore, it can be said that the log₁₀ [T] substantially corresponding to the linear regression equation represented by the equation (2a) can also be obtained for a ligand represented by the general formula (II) other than the ligand of Evaluation Example h1 and for ligands represented by the general formulae (I), (III), and (IV).

Accordingly, by using the linear regression equation represented by the equation (2a) as a regression model, among ligands represented by the general formulae (I) to (IV), a ligand with which log₁₀ [T] can be 5.0 or less and a curing time is shortened when such the ligand is used in a redox initiator including ketone peroxide and a cobalt organic acid salt in curing of a radically polymerizable resin at room temperature can be searched. Therefore, the ligand search method according to the present embodiment can be used for searching of a ligand of a curing accelerator that is any of ligands of the general formulae (I) to (IV), with which a curing time can be shortened, when a radically polymerizable resin is cured at room temperature.

### [Evaluation of extrapolation capability of generated regression model]

### (Derivation of linear regression equation)

A linear regression equation was derived in the same manner as in the above "(Calculation of logarithmic value of predicted curing time [T] (log₁₀ [T]))" using the ligand of Evaluation Example h1 as training data, and the ligand (L₅) of Reference Example 5, the ligand (L₇) of Reference Example 7, or the ligand (L₈) of Reference Example 8 as test data. The following linear regression equations represented by the equations (2a-1) to (2a-3) were derived from the physical property values xᵢ (NBO charge x₁ of a cobalt atom in a dormant species of a complex in which the ligand was coordinated to the cobalt atom, and percent buried volume x₂ of the ligand present within 3 Å of the cobalt atom in the dormant species of the complex in which the ligand was coordinated to the cobalt atom) acquired for the ligand (L₅) of Reference Example 5, the ligand (L₇) of Reference Example 7, and the ligand (L₈) of Reference Example 8, respectively. The equation (2a-1) is a linear regression equation for the ligand (L₅) of Reference Example 5, the equation (2a-2) is a linear regression equation for the ligand (L₇) of Reference Example 7, and the equation (2a-3) is a linear regression equation for the ligand (L₈) of Reference Example 8. ${log}_{10} \left[T\right] \left({x}_{i}\right) = 3.41227 {x}_{1} - 0.449646 {x}_{2} + 43.239$ (2a-1) ${log}_{10} \left[T\right] \left({x}_{i}\right) = 3.777831 {x}_{1} - 0.470965 {x}_{2} + 44.97$ (2a-2) ${log}_{10} \left[T\right] \left({x}_{i}\right) = 2.81583 {x}_{1} - 0.448084 {x}_{2} + 43.53$ (2a-3)

### (Calculation of logarithmic value (log₁₀ [T]) of predicted curing time [T])

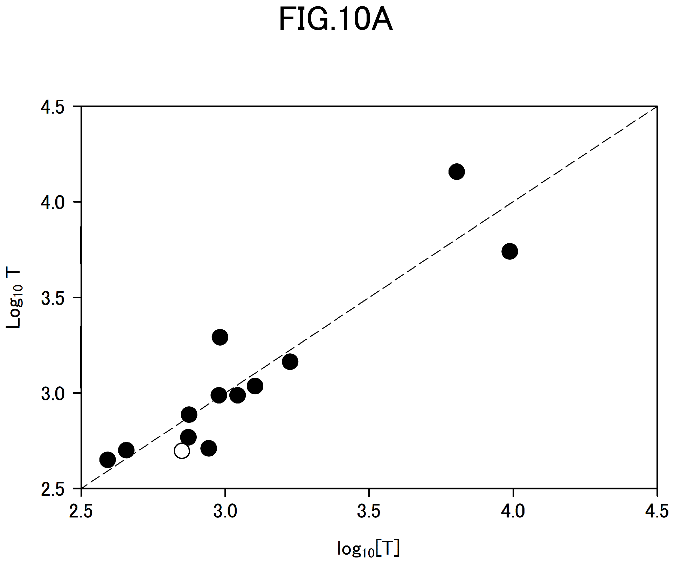

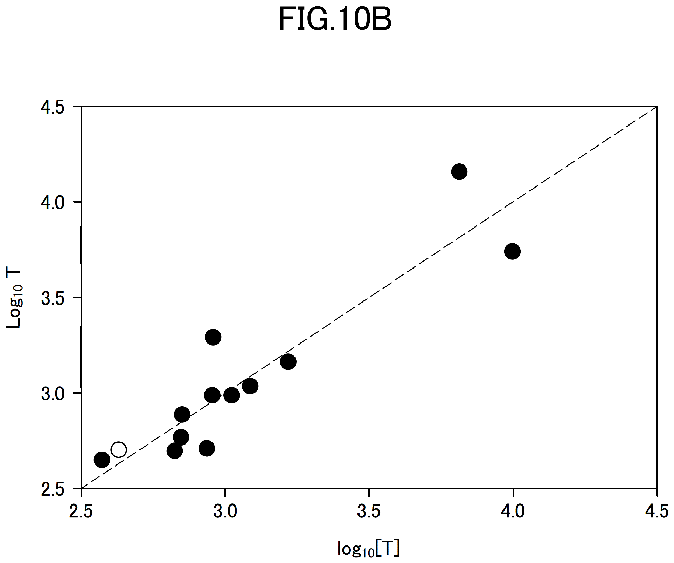

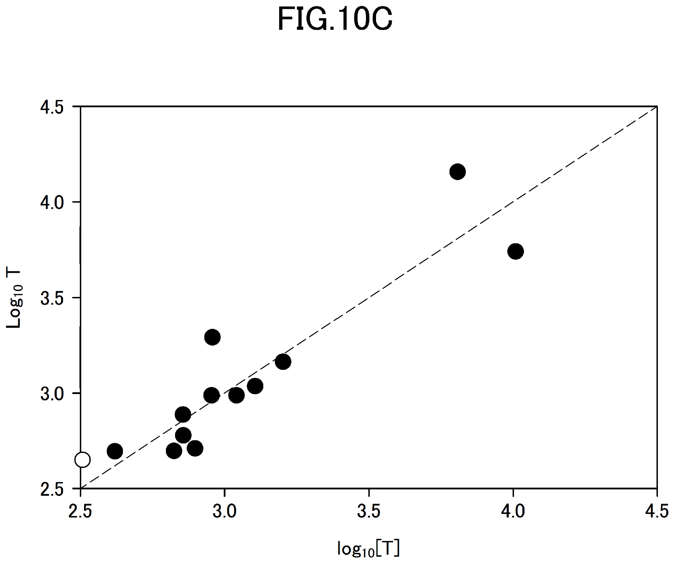

Using the derived equations (2a-1) to (2a-3), log₁₀ [T] was calculated for the ligand (L₅) of Reference Example 5, the ligand (L₇) of Reference Example 7, and the ligand (L₈) of Reference Example 8, respectively. The relationships between the log₁₀ [T] and the log₁₀ T using the ligands L₅, L₇, and L₈ of Reference Examples 5, 7, and 8 as the test data are presented in Figs. 10A to 10C, respectively. The results when the ligands (L₅), (L₇), and (L₈) were used as the test data are indicated as white circles. As illustrated in Figs. 10A to 10C, it was confirmed that the ligands (L₅), (L₇), and (L₈) were all ligands with which the log₁₀ [T] calculated by the equations (2a-1) to (2a-3) represented as the above linear regression equations representing linear regression equations was 5.0 or less. Accordingly, it can be said that sufficient prediction accuracy is ensure with a ligand capable of achieving a short curing time, such as the ligands (L₅), (L₇), and (L₈), and therefore the extrapolation capability of the generated regression model is high.

This application claims priority to Japanese Patent Application No. 2024-8833 filed before the Japan Patent Office on January 24, 2024, and Japanese Patent Application No. 2024-45903 filed before the Japan Patent Office on March 22, 2024, the entire contents of which are incorporated herein by reference.

### DESCRIPTION OF REFERENCE NUMERALS

1: ligand search system
100: regression model generation device
110, 210: physical property value acquisition unit
120: regression model generation unit
200: ligand search device
220: curing time calculation unit
230: ligand search unit
300: material database

## Claims

1. A method for searching for a ligand to be coordinated to cobalt, the method comprising:
causing a processor to execute processes including
searching for a ligand that is any of
a ligand represented by a general formula (I): 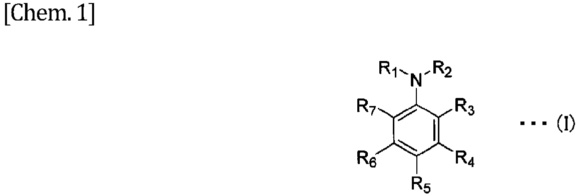 (in the formula, R₁ and R₂ are each independently a hydrogen atom or an alkyl group with 1 to 10 carbon atoms, and R₃ to R₇ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, or a halogen atom),
a ligand represented by a general formula (II): 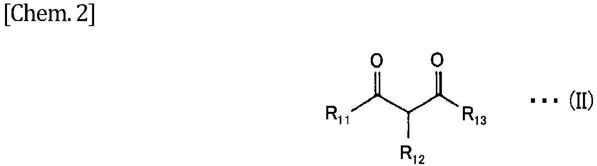 (in the formula, R₁₁ to R₁₃ are each independently a hydrogen atom, an alkyl group with 1 to 10 carbon atoms, an alkoxy group with 1 to 10 carbon atoms, an aryl group with 6 to 12 carbon atoms, an amino group, a carboxy group, a cyano group, or a halogen atom, and R₁₂ and R₁₃ may form a ring together),
a ligand represented by a general formula (III): 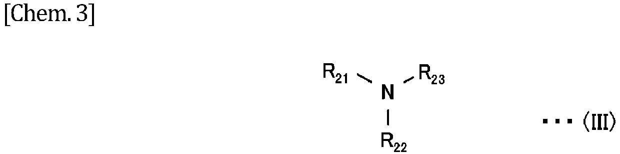 (in the formula, R₂₁ to R₂₃ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, or a halogen atom), and
a ligand represented by a general formula (IV): 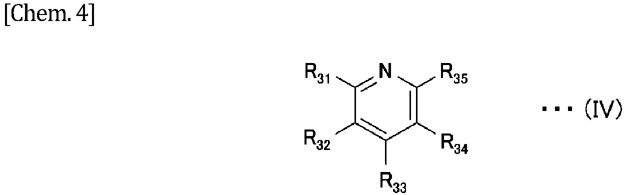 (in the formula, R₃₁ to R₃₅ are each independently a hydrogen atom, an alkyl group with 1 to 10 carbon atoms, an alkoxy group with 1 to 10 carbon atoms, an alkoxycarbonyl group with 2 to 11 carbon atoms, an aryl group with 6 to 12 carbon atoms, a hydroxy group, an amino group, a carboxy group, a cyano group, a nitro group, a formyl group, a halogen atom, or a pyridine ring),
the ligand being a ligand with which a logarithmic value of a predicted curing time [T] [sec] calculated by an equation (1) below is equal to or lower than a predetermined value, ${log}_{10} \left[T\right] = {α}_{0} + {α}_{1} {x}_{1} + {α}_{2} {x}_{2}$ (1) (in the equation, x₁ is an NBO charge of a cobalt atom in a dormant species of a complex in which the ligand is coordinated to the cobalt atom, x₂ is percent buried volume of the ligand present within 3 Å of the cobalt atom in the dormant species of the complex in which the ligand is coordinated to the cobalt atom, a₀, and a₁, and a₂ are coefficients).

2. The method for searching for the ligand according to claim 1,
wherein the equation (1) is an equation (2): ${log}_{10} \left[T\right] \left({x}_{i}\right) = 5.20950 {x}_{1} - 0.50360 {x}_{2} + 47.001$ (2), and
the predetermined value is 5.0.

3. The method for searching for the ligand according to claim 1 or 2,
wherein the dormant species of the complex in which the ligand represented by the general formula (I), (III), or (IV) is coordinated to the cobalt is,
on a basis of a cobalt (II) Werner-type complex in which two molecules of a carboxylate ligand as a bidentate ligand and two molecules of the ligand represented by the general formula (I), (III), or (IV) as a monodentate ligand are coordinated,
a dormant species of the Werner-type complex with cobalt (III), in which an OH radical or Me radical is coordinated instead of one of the two molecules of the ligand represented by the general formula (I), (III), or (IV).

4. The method for searching for the ligand according to any one of claims 1 to 3,
wherein the dormant species of the complex in which the ligand represented by the general formula (II) is coordinated to the cobalt is,
on a basis of a cobalt (III) Werner-type complex in which three molecules of the ligand represented by the general formula (II) as a bidentate ligand is coordinated,
a dormant species of the cobalt (III) Werner-type complex, in which a OH radical or Me radical is coordinated instead of one of the three molecules of the ligand represented by the general formula (II).
